# FASCICULE DE BREVET EUROPEEN

(11) **EP 1 528 107 B1**
(45) Date de publication et mention de la délivrance du brevet: **22.11.2006**
(21) Numéro de dépôt: 03024757.1
(22) Date de dépôt: 29.10.2003
(51) Int. Cl.: C12Q 1/68, G01N 33/574

(54) **Méthode pour le diagnostic de cancers**
Verfahren zur Diagnose von Krebs
Method for diagnosing cancers

(43) Date de publication de la demande: 04.05.2005
(73) Titulaire: Stroun, Maurice, 1208 Geneve (CH); Anker, Philippe, 1203 Geneve (CH)
(72) Inventeur: Stroun, Maurice, 1208 Geneve (CH); Anker, Philippe, 1203 Geneve (CH)
(74) Mandataire: Micheli & Cie SA

(56) Documents cités:
- EP-A- 1 158 055
- WO-A-97/35589
- WO-A-02/061148
- LO Y M D: "CIRCULATING NUCLEIC ACIDS IN PLASMA AND SERUM: AN OVERVIEW" ANNALS OF THE NEW YORK ACADEMY OF SCIENCES, NEW YORK ACADEMY OF SCIENCES, NEW YORK, NY, US, vol. 945, septembre 2001 (2001-09), pages 1-7, XP008019948 ISSN: 0077-8923
- ANKER PHILIPPE ET AL: "Circulating nucleic acids in plasma and serum as a noninvasive investigation for cancer: time for large-scale clinical studies?" 10 janvier 2003 (2003-01-10), INTERNATIONAL JOURNAL OF CANCER. JOURNAL INTERNATIONAL DU CANCER. 10 JAN 2003, VOL. 103, NR. 2, PAGE(S) 149 - 152 , XP002275956 ISSN: 0020-7136 * le document en entier *

## Description

La présente invention concerne une méthode de diagnostic, de pronostic et de suivi de l'évolution de divers types de cancers, par exemple après un traitement de chimiothérapie ou après une opération.

On sait que le diagnostic et le suivi des cancers sont effectués, à part l'observation et l'examen direct des tumeurs, par l'analyse de biopsies, ce qui implique soit une intervention chirurgicale soit un test invasif de type biopsie. Or, en plus du caractère désagréable, voire dangereux pour le patient, de telles méthodes, il a été constaté qu'elles pouvaient être peu précises.

On sait aussi que le diagnostic et le suivi des sources sont effectués pour l'analyse des composants ARN de l'enzyme télomérase présents dans le plasma ou le sérum sanguin (voir EP-A-1 158 055).

Le but de cette invention consiste donc à fournir une méthode de suivi de divers types de cancers qui remédie aux inconvénients précités et qui n'implique pas de test invasif sur les patients.

La méthode de diagnostic, de pronostic et de suivi des cancers, objet de l'invention, comprend la détection et la quantification de séquences bactériennes circulant sous forme libre dans le plasma ou le sérum. Il s'agit par exemple de séquences provenant d'Helicobacter dans le plasma de patients soupçonnés de souffrir d'un cancer de l'estomac ou d'un autre cancer digestif, de séquences de Chlamydia pneumoniae chez des personnes susceptibles d'avoir un cancer du poumon ou encore d'Helicobater pylori, de Chlamydia pneumoniae ou trachomatis, de Pseudomonas aeruginosa, de Salmonella typhimurium ou paratyphi, de Bifodobacterium, de Clostridium difficile, Mycobacterium tuberculosis, Streptococcus anginosus , de Citrobacter , ou d'Escherichia Coli, ou tout autre bactérie soupçonnée d'être cancérigène. Ces séquences bactériennes pourront également être cherchées dans les cellules du sang ou les cellules provenant de biopsies.

D'une manière générale, la méthode de diagnostic selon l'invention consiste à extraire l'ADN du plasma sanguin ou du sérum, des cellules du sang circulant ou au besoin d'une biopsie, à purifier cet ADN et à quantifier un gène bactérien avec ou sans amplification préalable. La portée de la présente invention s'étend bien entendu à toute technique d'extraction, de purification et d'amplification d'ADN, de même qu'à n'importe quelle méthode de détermination quantitative des gènes.

L'ADN est généralement amplifiée par PCR selon la méthode dite « Real TIME PCR », par exemple sur un appareil AB 5700, 7700 ou 9700 de la Société Applied Biosystems ou sur un « Light Cycler » de la Société Roche, ou encore sur n'importe quel appareil du même type.

De même, les gènes peuvent être détectés par la technique dite « Gene Array » sur puce.

Selon une variante, la quantification des gènes peut être obtenue sans amplification préalable par hybridisation avec des sondes spécifiques, ou encore par la méthode dite « SABRE », ou par n'importe quelle autre méthode de quantification de gènes ou d'expression de gènes adaptée à l'ADN.

On sait que les cancers sont caractérisés par un bouleversement du génome qui se traduit par des mutations, des délétions ou des amplifications de l'ADN, sans compter les altérations épigénétiques comme l'hyperméthylation de gènes promoteurs. Or, il a été trouvé que ces changements génétiques, tels que les mutations des gènes Ras, APC, P53, des altérations de microsatellites et des bouleversements épigénétiques pouvaient être présents sur l'ADN du plasma de patients atteints de diverses affections malignes. Certains auteurs ont aussi mis en évidence la présence en quantité augmentée de séquences virales provenant de virus Epstein Barr dans le sang de patients atteints de certains cancers, particulièrement le cancer nasopharyngien. Or, les présents inventeurs ont trouvé qu'il y a de l'ADN bactérien circulant dans le plasma sanguin de malades. De plus, lorsque des bactéries sont cultivées en contact avec des cellules humaines, il y a échange d'ADN. On retrouve de l'ADN bactérien dans les cellules humaines et des gènes humains dans les bactéries. Il a donc été postulé qu'il y a des séquences de gènes bactériens en quantité augmentée dans le cas de certains cancers, notamment le cancer de l'estomac, le cancer du colon ou le cancer du poumon.

La présente invention sera maintenant illustrée par les exemples qui suivent.

### Exemple 1

### Présence d'ADN bactérien dans le plasma sanguin humain.

Selon une application particulière de la méthode selon l'invention, on a cherché un gène de la bactérie *Chlamydia pneumoniae* dans le plasma de 4 malades soupçonnés d'être atteints de cancer du poumon et dans celui de 2 donneurs sains.

Un échantillon de sang (4ml ) a été prélevé sur EDTA . L'ADN a été extrait du sérum en utilisant un kit commercial, par exemple le « High Pure Viral Nucleic Acid Kit » de la maison Boehringer.

L'ADN ainsi extrait a été ensuite soumis à une amplification par PCR dans un appareil TaqMan 5700 de la firme AB Applied biosytems, appareil qui permet de quantifier un gène selon un sytème dit « Real Time PCR ».

Le gène amplifié était un gène de la bactérie *Chlamydia pneumoniae*. Les amplimères amorces étaient : sens 5'-ggaccttacctggacttgacatgt-3' et antisens 5'-ccatgcagcacctgtgtatctg-3'. Les sondes fluorescentes TaqMan inclues dans la séquence du produit synthétisé étaient 5'-tgacaactgtagaaatacagcctttccgcaaagg-3'.

Après amplification, la quantité relative du gène bactérien a été établie par la différence du nombre de cycles pour que la synthèse de chacun des amplimères devienne visible par fluorescence par rapport au nombre de cycles nécessaires pour une courbe de référence donnée par une quantité connue d'ADN de Chlamydia.

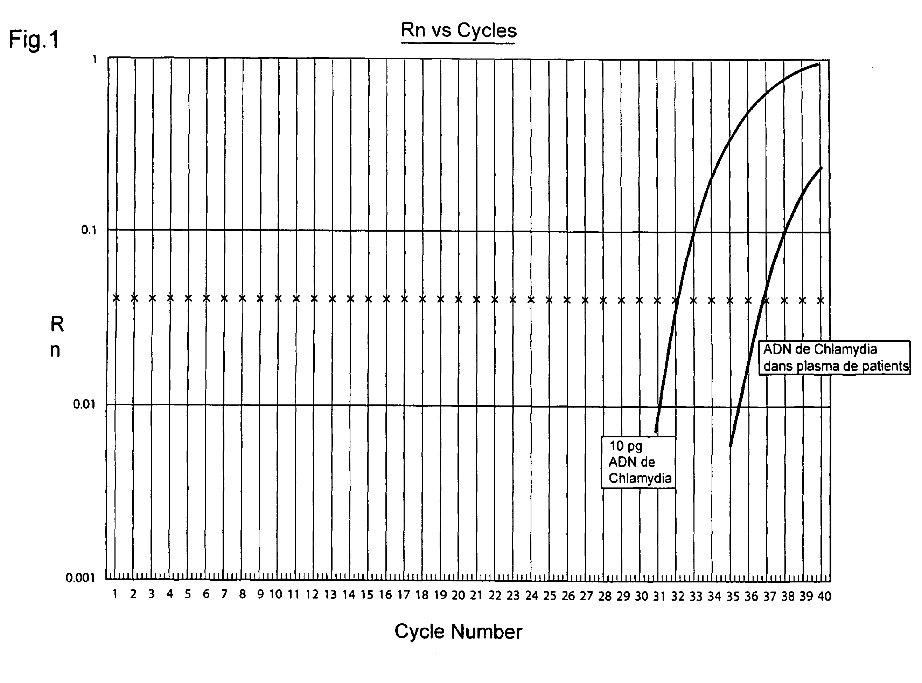

Le résultat de ces tests est résumé sur la Figure annexée.

Il ressort de ces résultats que l'on retrouve de l'ADN de la bactérie Chlamydia circulant librement dans le plasma sanguin de malades, alors qu'il est absent dans le plasma de contrôles sains. Cette méthode peut être utilisée tant pour la détection non invasive d'un cancer, en l'occurrence du poumon, que pour le suivi de l'évolution d'un cancer déjà détecté et le cas échéant traité.

### Exemple 2

### Présence d'ADN bactérien dans des cellules humaines après co-culture en présence de bactéries.

Des cellules de la lignée préleucémique HL 60 ont été cultivées en présence de bactéries, en l'occurrence de *Bacillus subtilis 168*.

Trois millions (3X10⁶) de cellules HL60 prises en pleine expansion sont placées dans des cupules (Millicell, sterilized Culture Plate Insert), de la maison Millipore. Ces cupules consistent en deux chambres distinctes, séparées par un filtre dont les pores ne dépassent pas 0.4 microns de diamètre. Dans l'autre chambre, on place environ 10⁸ bactéries. Le milieu de culture RPMI est celui dans lequel les cellules humaines sont en général clivées.

Cette co-culture se poursuit pendant 24, 48 et jusqu'à 72 heures, après lesquelles les cellules sont prélevées séparément. Un test de stérilité du surnageant et d'un lysat d'un aliquot de cellules HL60 est fait afin de s'assurer qu'aucune bactérie n'a contaminé les cellules humaines en traversant la membrane de séparation des deux chambres.

L'ADN des bactéries et des cellules humaines est extrait et purifié, puis ensuite soumis à une amplification par PCR dans un appareil TaqMan 5700 de la firme AB Applied biosytems, appareil qui permet de quantifier un gène selon un système dit « Real Time PCR ».

Les gènes amplifiés étaient un gène de la bactérie *Bacillus subtilis* et le gène K-ras humain. Les amplimères amorces étaient : B.subtilis-27 sens 5'- CAT CTA ATC AGC GGG TTA TTG G-3' et B.subtilis-98 antisens 5' - CGT GAC ATT CGA TAG TGA AAA ATC C -3' pour le gène de B.subtilis et et k-ras-13 sens 5' CTTGTGGTAGTTGGAGCTGGTG 3'et k-ras-94R antisens 5'-CATATTCGTCCACAAAATGATTCTG 3'pour le gène humain K-ras.

Les sondes fluorescentes TaqMan incluses dans la séquence du produit synthétisé étaient 5'- subtilis 52 - 6FAM - TGG ATG TAA ACC ACT ATT T -3' pour le gène de B. subtilis et k-ras-50T et 5' CCTTGACGATACAGCTAA-3' pour le K-ras. Après amplification, la quantité relative du gène bactérien a été établie par la différence du nombre de cycles pour que la synthèse de chacun des amplimères devienne visible par fluorescence par rapport au nombre de cycles nécessaires pour une courbe de référence donnée par une quantité connue d'ADN de B.Subtilis, et de même la quantité d'ADN humain est établi d'après une courbe de référence K-ras donnée par des quantités connues d'ADN humain.

Le résultat d de ces tests est résumé sur le tableau ci-après.

| HEURES DE CULTURE | Copies d'ADN bactérien/10 5 cellules humaine | Copie d'ADN humain par 10 6 bactéries |
|---|---|---|
| 24h expérience 1 | 2 copies/10 5 cellules | 23 copies/10 6 bactéries |
| 24h expérience 2 | 1.5 copies/10 5 cellules | 18 copies/10 6 bactéries |
| 48h expérience 1 | 53 copies/ 10 5 cellules | 400 copies/10 6 bactéries |
| 48h expérience 2 | 4 copies /10 5 cellules | 5 copies/10 6 bactéries |
| 72h expérience 1 | 29 copies /10 5 cellules | 240 copies/10 6 bactéries |
| 72h expérience 2 | 116 copies /10 5 cellules | 318 copies/10 6 bactéries |

Il ressort de ces résultats que lorsque des cellules humaines et des bactéries sont mises en présence comme cela peut être le cas lors d'une septicémie ou d'une péritonite, par exemple, il peut y avoir échange d'ADN entre les cellules procaryotes et les cellules eucaryotes. Quand on constate que le gène ras (dont une mutation ponctuelle est fortement cancérigène) peut être pris par une bactérie, on comprend mieux le rôle qu'elles peuvent éventuellement jouer lors de l'initiation ou du développement de certains cancers.

## Revendications

1. Méthode pour le diagnostic, le pronostic et le suivi de cancers comprenant la détection de la présence d'ADN bactérien présent dans le plasma ou le sérum sanguin, dans les cellules du sang ou dans des tumeurs, puis sa quantification.

2. Méthode selon la revendication 1, **caractérisée par le fait qu**'on extrait l'ADN, qu'on le purifie et qu'on quantifie un ou plusieurs gène(s) et un ou plusieurs gène(s) unique, puis qu'on détermine leurs quantités respectives.

3. Méthode selon la revendication 1 ou la revendication 2 , **caractérisée par le fait que** les génes bactériens cherchés proviennent d'*Helicobater pylori,* de *Chlamydia pneumoniae* ou *trachomatis,* de *Pseudomonas aeruginosa,* de *Salmonella typhimurium* ou *paratyphi,* de *Bifodobacterium,* de *Clostridium difficile, Mycobacterium tuberculosis, Streptococcus anginosus ,* de *Citrobacter ,* ou d'*Escherichia Coli,* ou tout autre bactérie soupçonnée d'être cancérigène.

4. Méthode selon l'une des revendications 1 à 3, **caractérisée par le fait que** les gènes sont quantifiés sans amplification préalable.

5. Méthode selon l'une des revendications 1 à 3, **caractérisée par le fait que** les gènes sont détectés et quantifiés par « gene array » sur puce.

6. Méthode selon l'une des revendications 1 à 3, **caractérisée par le fait que** l'ADN est amplifié et la quantité de l'ADN bactérien est estimé par « Real Time » PCR qui établit la quantité par rapport à une référence d'ADN connue grâce à la différence du nombre de cycles PCR nécessaires pour que la synthèse de chacun des amplimères devienne visible par fluorescence.

## Claims

1. Method for the diagnosis, prognosis, and tracking of cancers, comprising the detection of the presence of bacterial DNA in blood plasma or serum, in the blood cells or in tumours, then its quantification.

2. Method of claim 1, **characterised in that** the DNA is extracted, purified, one or several genes and one or several unique genes are quantified and then their respective quantities are determined.

3. Method of claim 1 or claim 2, **characterised in that** the bacterial genes being sought come from *Helicobacter pylori, Chlamydia pneumoniae* or *trachomatis, Pseudomonas aeruginosa, Salmonella typhimurium* or *paratyphi, Bifidobacterium, Clostridium difficile, Mycobacterium tuberculosis, Streptococcus anginosus, Citrobacter* or *Escherichia coli* or any other bacteria suspected to be carcinogenic.

4. Method of one of claims 1 to 3, **characterised in that** the genes are quantified without a preliminary amplification.

5. Method of one of claims 1 to 3, **characterised in that** the genes are detected and quantified by gene arrays on chips.

6. Method of one of claims 1 to 3, **characterised in that** the DNA is amplified and the amount of bacterial DNA estimated by real-time PCR establishing the amount relative to a known DNA reference via the difference in the number of PCR cycles needed for the synthesis of each of the amplimers to become visible by fluorescence.

## Patentansprüche

1. Verfahren zur Diagnose, Prognose und Nachsorge von Krebsarten, den Nachweis des Vorhandenseins bakterieller DNA im Blutplasma oder Blutserum, in den Blutzellen oder in Tumoren und danach ihre Quantifizierung umfassend.

2. Verfahren nach Anspruch 1, **dadurch gekennzeichnet, dass** die DNA extrahiert und gereinigt wird und dass ein oder mehrere Gene und ein oder mehrere einzigartige Gene quantifiziert, dann ihre jeweiligen Mengen bestimmt werden.

3. Verfahren nach Anspruch 1 oder Anspruch 2, **dadurch gekennzeichnet, dass** die gesuchten bakteriellen Gene von *Helicobacter pylori, Chlamydia pneumoniae* oder *trachomatis, Pseudomonas aeruginosa, Salmonella typhimurium* oder *paratyphi, Bifidobacterium, Clostridium difficile, Mycobacterium tuberculosis, Streptococcus anginosus, Citrobacter* oder *Escherichia coli* oder jedweden anderen Bakterien stammen, die verdächtigt werden, krebserregend zu sein.

4. Verfahren nach einem der Ansprüche 1 bis 3, **dadurch gekennzeichnet, dass** die Gene ohne vorausgehende Amplifizierung quantifiziert werden.

5. Verfahren nach einem der Ansprüche 1 bis 3, **dadurch gekennzeichnet, dass** die Gene durch "Genarrays" auf Chips nachgewiesen und quantifiziert werden.

6. Verfahren nach einem der Ansprüche 1 bis 3, **dadurch gekennzeichnet, dass** die DNA amplifiziert und die Menge der bakteriellen DNA durch Echtzeit-PCR geschätzt wird, die die Menge im Verhältnis zu einer bekannten Bezugs-DNA dank des Unterschieds in der Zahl von PCR-Zyklen festlegt, die erforderlich sind, damit die Synthese jedes der Amplimere durch Fluoreszenz sichtbar wird.
